(19) Europäisches Patentamt
European Patent Office
Office européen des brevets

(11) Numéro de publication: 0 174 879
B1

(12) # FASCICULE DE BREVET EUROPEEN

(45) Date de publication du fascicule du brevet: 19.09.90

(21) Numéro de dépôt: 85401433.9

(22) Date de dépôt: 12.07.85

(51) Int. Cl.$^5$: C 07 H 21/00, C 12 Q 1/68, C 07 K 3/12

(54) **Polynucléotides liés de façon covalente à un support solide, et procédé pour leur obtention.**

(30) Priorité: 12.07.84 FR 8411101

(43) Date de publication de la demande:
19.03.86 Bulletin 86/12

(45) Mention de la délivrance du brevet:
19.09.90 Bulletin 90/38

(84) Etats contractants désignés:
AT BE CH DE FR GB IT LI LU NL SE

(56) Documents cités:
EP-A- 27 631
EP-A-0 063 879
EP-A-0 117 440
WO-A-85/01051
DE-A-2 841 414

ZEITSCHRIFT FÜR CHEMIE, volume 23, no. 9, septembre 1983, pages 317-326; A. ROSENTHAL et al.: "Chemische Synthese von DNA-Sequenzen"

Nucl. Acids Res. Vol. 16(1988)P.1618
Chem. Ber. 107(1974) p. 24-33

Affinity Chromotography (1978) p.177, 183, 261-5, 270, 384

(73) Titulaire: INSTITUT PASTEUR
25-28, rue du Docteur Roux
F-75724 Paris Cédex 15 (FR)
(73) Titulaire: CENTRE NATIONAL DE LA RECHERCHE SCIENTIFIQUE (CNRS)
15, Quai Anatole France
F-75007 Paris (FR)

(72) Inventeur: Huynh-Dinh, Tam
32, rue Paul Déroulède
F-78290 Croissy Sur Seine (FR)
Inventeur: Pochet, Sylvie
201, rue Lecourbe
F-75015 Paris (FR)
Inventeur: Igolen, Jean
4, rue Croix Mathurine
F-78320 Le Mesnil St-Denis (FR)

(74) Mandataire: Peaucelle, Chantal
S.C. Ernest Gutmann - Yves Plasseraud 67, boulevard Haussmann
F-75008 Paris (FR)

EP 0 174 879 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

L'invention concerne des polynucléotides, plus particulièrement des fragments d'oligonucléotides, fixés de façon covalente à un support solide, un procédé de préparation de ces polynucléotides — ou oligonucléotides — supportés, des produits intermédiaires utiles pour leur fabrication, des procédés pour leur obtention, ainsi que leurs applications.

L'invention concerne plus particulièrement encore des techniques permettant la production de sondes à l'aide des polynucléotides plus particulièrement des fragments d'oligonucléotides ci-dessus. Il s'agit plus spécialement de sondes mettant en oeuvre des séquences d'acides nucléotidiques de petite taille, notamment des oligonucléotides pouvant comporter moins de 50 ou même moins de 20 nucléotides, ces sondes étant supportées par un support solide ou fixées à celui-ci.

Les techniques de détection basées sur l'hybridation entre une séquence déterminée de nucléotides utilisée en tant que sonde et une séquence de nucléotides complémentaires de ceux de la sonde, éventuellement contenue dans une composition renfermant des acides nucléiques, sont maintenant bien connues du spécialiste. Ces techniques restent cependant difficiles à mettre en oeuvre et ne sont susceptibles de conduire à des résultats reproductibles qu'entre les mains de spécialistes très entraînés, et ce plus particulièrement lorsque c'est la sonde qui est fixée à un support et que le mélange d'acides nucléiques contenant éventuellement le fragment de nucléotides à détecter est amenée et maintenue au contact de ces sondes dans des conditions permettant l'hybridation.

La fabrication de sondes supportées fait en général intervenir des réactions de fixation des séquence-sondes au support. Ces fixations, éventuellement renforcées par l'usage de réactifs chimiques (par exemple carbodiimide ou réactifs de diazotation), s'effectuent cependant en général en des points ou sites répartis au hasard le long des séquences nucléotidiques en cause. Il en résulte cependant que seules les parties non fixées de la sonde sont exposées, dans des conditions permettant leur hybridation éventuelle, avec les séquences nucléotidiques complémentaires éventuellement contenues dans un mélange.

On comprendra par conséquent que les modes de fixation classiques cessent rapidement d'être applicables à des séquences sondes de faible longueur.

Dans la demande WO85/01051 au nom de Molecular Biosystems, publiée le 14 mars 1985, on décrit des supports polymères pour la synthèse d'oligonucléotides.

Cette synthèse est réalisée comme suit (voir page 6, lignes 4 à 19 de la demande internationale):

a — on choisit un support polymère,

b — on fixe une amorce à ce support, une partie de l'amorce possédant un ou plusieurs substituants oxydables,

c — on protège les substituants oxydables avec des groupes de blocage,

d — on protège les groupes réactifs du support polymère avec des groupes de blocage,

e — on condense des nucléotides sur l'amorce pour synthètiser un oligonucléotide,

f — on élimine les groupes protecteurs des substituants oxydables après synthèse complète de l'oligonucléotide,

g — on oxyde sélectivement les substituants oxydables à l'aide d'un agent oxydant,

h — simultanément, ou après l'étape g, on traite l'oligonucléotide avec une base et on récupère l'oligonucléotide.

L'amorce peut être un ribonucléoside lié au support polymère par sa base.

Selon l'exemple 1 ou 3, la base de l'amorce est une adénine. Elle est reliée au support par l'intermédiaire d'un groupe dodécylamine fixé en position 6.

Les moyens décrits correspondent à des oligo et polynucléotides supportés différents de ceux de l'invention et en particulier ne permettent pas d'accéder avec le même type d'amorce fixée à un support à des oligonucléotides monobrins ou doubles brins supportés.

Dans Chem. Ber. 107 (1974), 24—33, on décrit la synthèse chimique de déoxyoligonucléotides.

Le premier déoxynucléotide, dont la position 3' est bloquée par un groupement acétyle, est fixé par l'intermédiaire de son groupe phosphate en position 5' sur une amorce.

Le groupement de blocage en position 3' est alors éliminé et un nouveau nucléoside bloqué en position 3' est fixé sur le groupe —OH libéré de l'amorce par l'intermédiaire d'un groupe phosphate en 5'. La synthèse est poursuivie et lorsque le nombre désiré de nucléotides a été fixé, l'oligonucléotide est détaché du support polymère à l'aide d'un mélange acide trifluoroacétique/$CH_2Cl_2$, ce qui élimine également les groupements protecteurs de l'amorce.

Le groupe 5' phosphate lié à l'amorce est ensuite libéré par oxydation à l'aide de periodate et bêta élimination avec de l'ammoniaque.

La DAS 2841414, la demande EP 027631 et l'ouvrage Affinity Chromatography, 1978, pages 1, 77, 183, 261—270 et 384 décrivent des composés renfermant un cycle adénine uni par liaison covalente à un support.

L'invention à pour but de fournir des séquences nucléotidiques supportées de très faible taille, celles-ci pouvant soit elles-mêmes intervenir dans les réactions d'hybridation avec des séquences nucléotidiques complémentaires dans des conditions classiques, soit servir d'amorces à la fixation de séquences plus longues à ce même support.

L'invention concerne également un procédé d'obtention de ces polynucléotides supportés, procédé

qui résulte de l'adaptation appropriée des techniques de synthèse d'oligonucléotides en phase solide, par exemple les méthodes dites aux phospho-triesters ou aux phosphoramidites, dans des conditions telles que l'on puisse en définitive disposer d'oligonucléotides non protégés, caractérisés par des séquences déterminées, liés de façon covalente à un support solide.

L'invention concerne tout d'abord une molécule supportée qui, en quelque sorte, peut être considérée comme l'amorce possible de la réalisation d'un oligonucléotide ou polynucléotide supporté. Cette molécule supportée comporte dans sa structure au moins un groupe nucléoside ou nucléotide fixé de façon covalente à un support solide, le site de fixation au groupe nucléoside intervenant au niveau de l'hétérocycle entrant dans la constitution dudit groupe nucléoside. Ce mode de liaison laisse alors libre les hydroxyles du sucre et permet de constituer des chaînes oligonucléotidiques (ADN ou ARN) dans le sens 3' → 5' et/ou dans le sens 5' → 3'.

L'invention concerne en outre les oligonucléotides ou polynucléotides supportés qui notamment peuvent être formés à partir de la molécule supportée ci-dessus, par la mise en jeu des techniques antérieurement connues d'allongement de chaînes de nucléotides en phase solide, par exemple dans des conditions qui ont été décrites dans les articles (1) à (6) de la bibliographie figurant à la fin de cette description. Le support permet également la synthèse enzymatique de fragments d'ADN ou d'ARN avec une ADN ou ARN ligase comme décrit dans les références (13) et (14) (voir bibliographie).

On observera cependant qu'en substituant la molécule supportée définie ci-dessus aux produits résultant de l'ancrage d'un premier nucléoside (désoxyribonucléoside ou ribonucléoside) sur un support fonctionnalisé approprié dans les procédés d'allongement de la chaîne nucléotidique selon les méthodes classiques, il sera possible au moment de la réalisation des opérations de déprotection des séquences oligonucléotidiques synthétisées, d'obtenir la séquence nucléotidique ainsi synthétisée libre de tout groupe protecteur, mais toujours encore liée de façon covalente au support solide. En cela, le procédé de l'invention se distingue des procédés antérieurs de synthèse d'oligonucléotides en phase solide, dont l'étape finale de déprotection ou élimination des groupes protecteurs, notamment par un traitement acide, basique ou ces deux traitements, implique également l'élimination du support.

De préférence, le site de fixation au support du groupe nucléosidique ou du nucléotide terminal de l'oligonucléotide ou polynucléotide se situe au niveau du sommet C—4 du cycle pyrimidine ou du sommet C—6 du cycle purine, selon le cas, dudit groupe nucléoside ou dudit nucléotide terminal.

De préférence, la fixation covalente desdits groupes nucléosides ou nucléotides au support du genre en question se fait par l'intermédiaire d'un bras dont la longueur correspond de préférence à celle d'une chaîne carbonée ayant de 4 à 20 atomes de carbone.

Il est clair que la longueur de cette chaîne ne constitue pas en soi un facteur critique essentiel, si ce n'est qu'un bras trop court serait éventuellement incompatible avec l'exposition complète ultérieure de l'oligonucléotide supporté à des acides nucléiques contenant une séquence complémentaire de nucléotides venant à leur contact, à l'occasion d'opérations d'hybridation, et que des chaînes trop longues seraient d'une trop grande flexibilité pouvant également interférer avec la réalisation d'une hybridation dans des conditions semblables. En particulier, les chaînes risqueraient de se replier.

Les oligonucléotides ou polynucléotides fixés sur un support solide, selon l'invention, sont caractérisés en ce qu'ils comportent au moins une structure de formule:

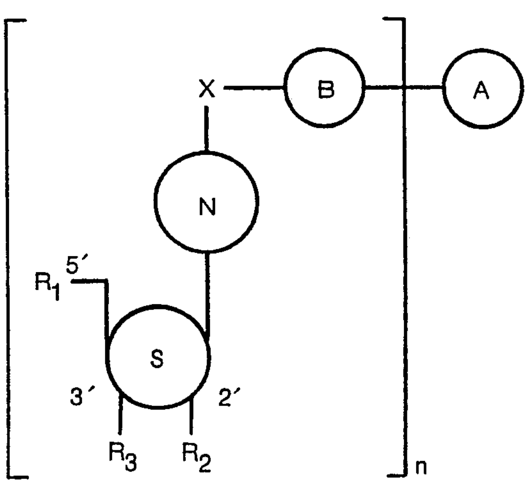

dans laquelle:

N représente une base, telle que uracile, méthyl-5-uracile (thymine), guanine, xanthine, méthyl-5-cytosine, cytosine, amino-2-adénine, hypoxanthine;

S représente un motif ribose ou désoxy-2'-ribose avec $R_1$ représentant un groupe OH, phosphate ou encore oligo ou polynucléotide, $R_2$ un atome d'hydrogène ou un groupe OH et $R_3$ un groupe OH, phosphate ou encore oligo ou polynucléotide, les chiffres (2'), (3') et (5') correspondant aux sommets, numérotés de façon classique, des sucres en question, les séquences de l'oligo ou polynucléotide pouvant être

complémentaires et formant un double brin ou l'oligonucléotide fixé sur l'amorce pouvant être replié en boucle en formant un double brin et étant fixé sur l'amorce par l'un des nucléotides de la boucle,

X représente un groupe divalent NH ou O fixé sur la base N au niveau des sommets 4 ou 6 respectivement pour une base pyrimidique et pour une base purique,

B représente une chaîne carbonée de 4 à 20 atomes de carbone,

A représente un support solide,

n représente le nombre de chaînes fixées sur un même support A, ces chaînes pouvant être différentes les unes des autres et fixées par des bras identiques ou différents sur le support, sous réserve que lorsque N représente une adénine et l'un des groupes $R_1$ et $R_3$ représente un groupe —OH et X représente NH, B soit différent d'un radical dodécylamine.

Un bras B préféré est constitué par une chaîne carbonée comprenant de préférence de 4 à 20 atomes de carbone, dont certains peuvent le cas échéant être remplacés par des hétéroatomes tels que O, N ou S.

De préférence, X—B correspond à une chaîne dérivée d'une chaîne bifonctionnelle, telle que l'éthylène diamine, le diamino 1—6 hexane, le diamino 1—10 décane, la spermine, des diéthylène-glycols ou dialcoylène-glycols.

Selon une autre vairante préférée, le bras B renferme un groupe M'' choisi parmi une fonction amide, éther, sulfure, uréthane ou encore un groupe —NH— ligand, ce groupe M'' résultant de la réaction d'une part du groupe réactif M, situé à l'extrémité d'un bras fixé à un nucléotide ou à un nucléoside, avec d'autre part un groupe réactif M' fixé sur le support solide A.

Il est entendu que l'expression "support solide" recouvre tout support qui peut rester insoluble dans au moins un milieu déterminé susceptible de contenir des acides nucléiques en solution. De nombreux supports peuvent être utilisés.

Dans le cas de bras B formé d'une chaîne carbonée, le support solide A est plus spécialement choisi parmi ceux compatibles à la fois avec les solvants organiques et les tampons aqueux mis en oeuvre pour la préparation des molécules supportées.

En variante, l'invention vise des oligonucléotides ou polynucléotides fixés sur un support A tels qu'obtenus par allongement de la chaîne nucléotidique en positions 3' et 5' d'une amorce répondant à la structure:

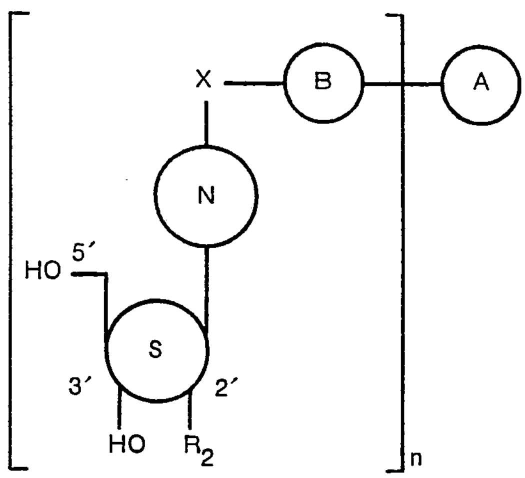

dans laquelle:

N représente une base, telle que uracile, méthyl-5-uracile, guanine, xanthine, méthyl-5-cytidine, cytidine, thymidine, amino-2-adénine, inosine;

S représente un motif ribose ou désoxy-2'-ribose avec $R_2$ représentant un atome d'hydrogène ou un groupe OH;

X représente un groupe divalent NH ou O fixé sur la base N au niveau des sommets 4 ou 6 respectivement pour une base pyrimidique et pour une base purique,

B représente une chaîne carbonée de 4 à 20 atomes de carbone,

A représente un support solide,

n représente le nombre de chaînes fixées sur un même support A, ces chaînes pouvant être différentes les unes des autres et fixées par des bras identiques ou différents sur le support, l'allongement des chaînes étant suivi le cas échéant d'une déprotection des nucléotides ou des nucléosides.

Il s'agit par exemple de polymères, de silice ou de billes de verre, ces supports ayant cependant, le cas échéant, fait l'objet d'un traitement préalable (fonctionnalisation), leur conférant des groupements fonctionnels susceptibles d'être mis en jeu dans une réaction de fixation d'une molécule distincte, soit directement, soit par l'intermédiaire d'un bras. Des supports de ce type sont disponibles dans le commerce. Il s'agit par exemple des gels du type polyacrylmorpholide, par exemple ceux commercialisés sous la désignation "ENZACRYL K—2".

Lorsque le bras B est terminé par un radical fonctionnel M, le support solide A ne doit pas forcément

répondre à ces exigences et est choisi parmi n'importe quel type de support existant sur le marché tels que les agaroses, notamment ceux commercialisés sous la marque Sépharose, les polyacrylamides, polystyrènes ou cellulose.

L'invention vise également un procédé de préparation d'oligonucléotides et de polynucléotides tels que définis ci-dessus.

Ce procédé est caractérisé en ce qu'il comprend la réaction

a) du sommet 6 ou 4 respectivement d'une base purique ou pyrimidique d'un nucléoside ou d'un nucléotide, ce sommet étant activé par un dérivé hétérocyclique ou par l'intermédiaire d'un chlorure d'acide, avec

b) un groupe terminal $NH_2$ ou OH d'un bras B, tel que défini dans la revendication 1, fixé à un support solide,

c) la synthèse de la chaîne nucléotidique en 3' et/ou 5' selon les techniques classiques, suivie le cas échéant d'une déprotection des nucléotides et nucléosides.

Selon une autre variante, la synthèse des oligonucléotides et polynucléotides de l'invention comprend:

1) la réaction du sommet activé 6 ou 4 respectivement d'une base purique ou pyrimidique située dans une position quelconque dans un oligonucléotide synthétisé au préalable, avec un groupe terminal réactif d'une chaîne bifonctionnelle, puis

2) la réaction du nucléoside ou du nucléotide fonctionnalisé obtenu avec un support solide approprié.

De préférence, cette variante est effectuée selon la réaction illustrée par le schéma suivant:

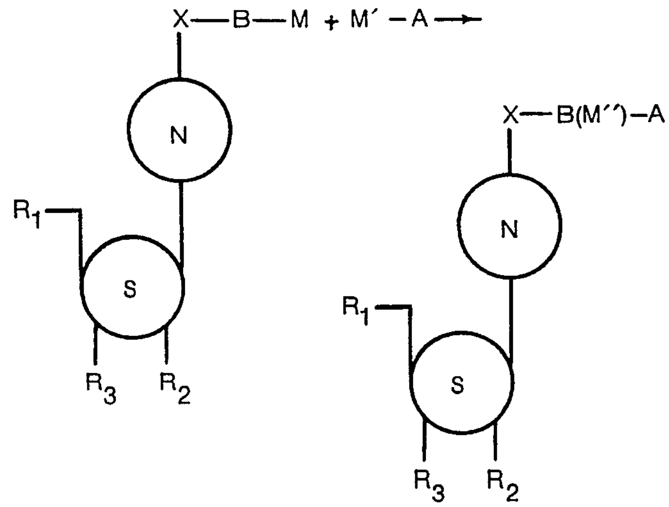

dans lequel $R_1$ à $R_3$, S, N, X, B, M'' et A sont tels que définis ci-dessus, M est choisi parmi $-NH_2$, —SH, —OH, —COOH ou NH-ligand, et M' représente, respectivement, compte tenu des significations ci-dessus de *M* un groupe —COOH, —SH, —OH ou $NH_2$, B (M'') signifiant que B renferme le groupe fonctionnel M'' qui résulte de la réaction de M avec M' défini plus haut.

Selon un aspect de grand intérêt, dans le procédé, ou la variante définie ci-dessus, on fixe un oligonucléotide double brin fonctionnalisé en position 4 ou 6 respectivement d'une base pyrimidique ou purique sur un support M'A.

La liaison entre M' et A' est réalisée selon les techniques classiques, illustrées en particulier par les schémas suivants:

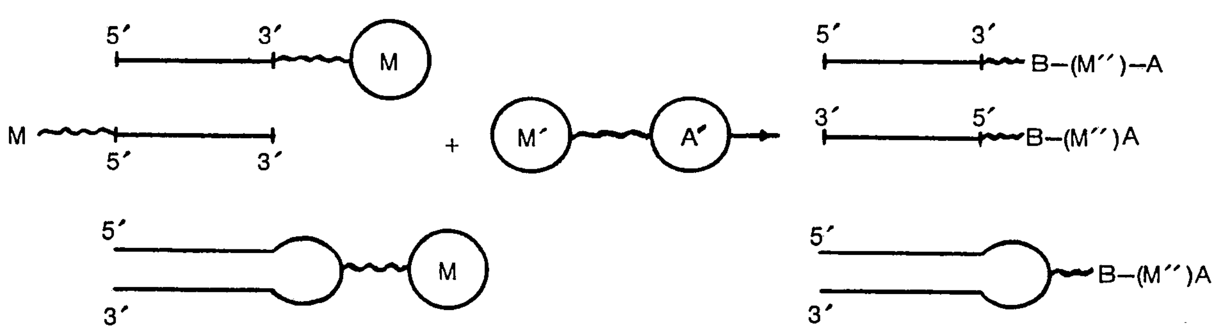


La synthèse des oligonucléotides comportant un bras M'' est effectuée chimiquement à l'aide des nucléosides modifiés X et Y décrits dans la demande de brevet 8413096 du 22 août 1984 aux noms de l'Institut Pasteur et du CNRS. En incorporant les nucléosides modifiés en 3', 5' ou en un ou plusieurs sites

quelconques de la séquence, on fait réagir à la fin de la synthèse une chaîne X ~ B ~ M' comme illustré dans les exemples (II.2 et III.1).

On s'assurera naturellement que le fragment d'oligonucléotide porte un bras avec une extrémité réactive M, pour que la liaison covalente M'' puisse s'établir avec le support portant la fonction M' activée.

D'une manière avantageuse, cette variante permet de greffer la séquence d'ADN (ou ARN) préparée à l'aide d'un synthétiseur automatique et purifiée manuellement par CLHP ou électrophorèse avant d'être mise en réaction avec une fonction activée du support, ce qui conduit à l'obtention d'un fragment d'ADN purifié lié de façon covalente au support solide.

On remarquera également qu'il est possible de mettre 2 ou plusieurs fragments d'oligonucléotide monobrin ou double brin en une seule réaction chimique.

Un mode d'activation préféré du sommet 6 ou 4 respectivement de la base purique ou pyrimidique du nucléotide comprend la fixation sur ce sommet d'un dérivé hétérocyclique, du type triazole, nitro-triazole, tétrazole, par exemple selon l'une des techniques décrites dans les références bibliographiques indiquées à la fin de cette description. Des nucléosides (ou le nucléotide terminal d'un oligonucléotide) ainsi activés peuvent alors être mis en réaction avec un support fonctionnalisé porteur de groupes fonctionnels terminaux $NH_2$, par exemple pour produire la réaction représentée par le schéma qui suit:

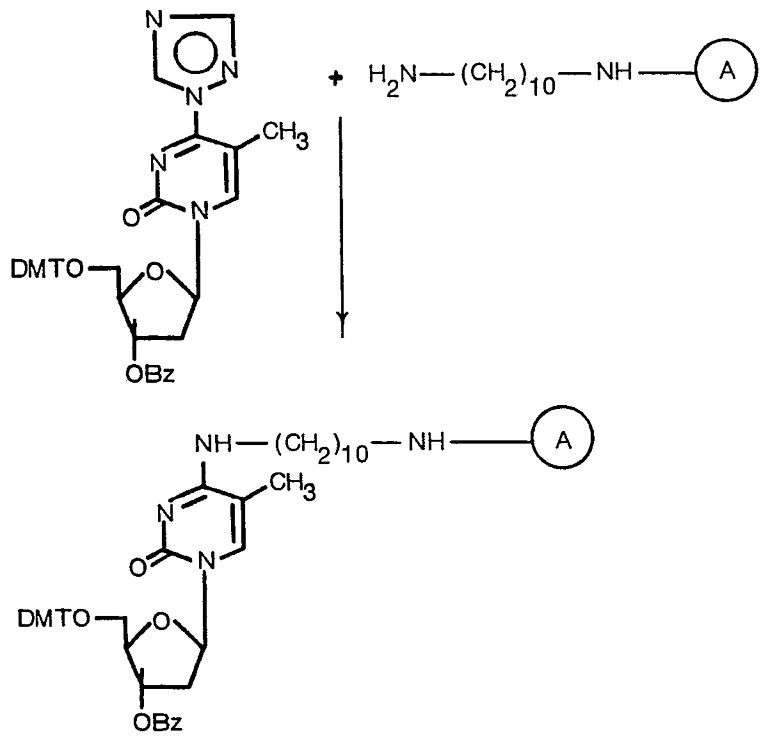

Dans les formules chimiques qui précèdent:
A représente un support solide,
Bz représente un groupe benzoyle et
DMT représente un groupe diméthoxytrityle.

Il va de soi que tout autre type de réaction chimique mettant en jeu l'activation préalable des mêmes sommets des bases hétérocycliques, ou de tout autre groupe éventuellement préalablement fixé sur ces bases, et la réaction du groupe activé avec le support dans les conditions sus-indiquées peut être envisagée. Pour mémoire, on rappellera par exemple les procédés d'activation qui ont été décrits des mêmes sommets des bases hétérocycliques, par l'intermédiaire d'un chlorure d'acide (référence bibliographiques (10) et (11).

Le procédé selon l'invention permet donc de réaliser nucléotide par nucléotide (ou groupe de nucléotides par groupe de nucléotides) des oligonucléotides de synthèse finalement exempts de tout groupe protecteur et liés de façon stable à un support solide. Il a été constaté que les traitements chimiques basiques et acides classiquement utilisés pour la déprotection des séquences oligonucléotidiques ne modifient pas la stabilité de la séquence nucléotidique complètement déprotégée sur le support. Les supports portant ainsi des séquences hybridables peuvent être mis en oeuvre dans toutes applications mettant en jeu un tel type de réaction.

La technique selon l'invention permet donc, non seulement la réalisation de séquences oligonucléotidiques de faible taille, liées de façon covalente et stable à un support, mais encore d'acides nucléiques plus importants, également liés à ce même support.

Il peut également être envisagé de fixer sur un même support plusieurs oligonucléotides ayant des

séquences nucléotidiques distinctes, notamment après activation des bases de leurs nucléotides d'extrémités respectives et fixation à des extrémités de bras distincts portés par ce même support.

Comme indiqué ci-dessus, les fragments d'ADN ou d'ARN peuvent être synthétisés sur la molécule supportée par voie enzymatique à l'aide d'une ARN ligase selon le schéma:

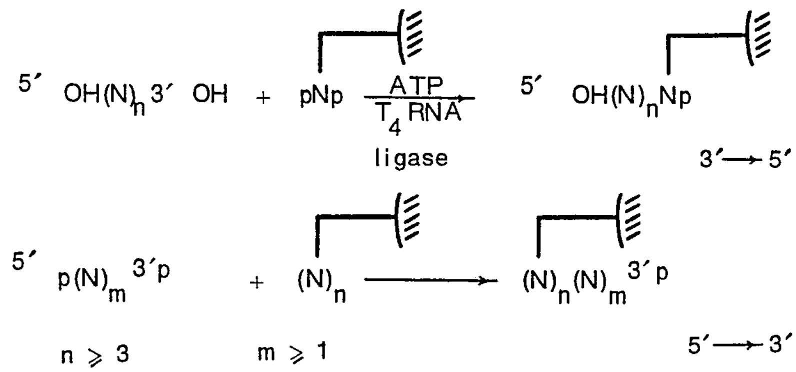


On notera en outre, que l'invention peut être mise en oeuvre de façon à autoriser la séparation finale de l'oligonucléotide formé du support par des réactions chimiques ou enzymatiques décrites dans la littérature (12). La séparation peut intervenir, soit en un site spécifique à proximité du support ou à l'extrémité du bras, soit dans un site prévu dans la chaîne nucléotidique elle-même.

Par exemple, il peut être envisagé que les premiers éléments de la séquence nucléotidique soient formés de ribonucléotides, les suivants étant alors tous constitués par des désoxyribonucléotides. La séparation entre la chaîne de désoxyribonucléotides et le support pourra alors être réalisée de façon en soi connue, par exemple par un traitement au moyen de réactifs chimiques, ou d'une ribonucléase.

Les oligonucléotides ou les polynucléotides mono brin ou double brin, fixés sur un support solide, tels que définis ci-dessus, sont utilisables pour l'élaboration de sondes pour la détection de séquences complémentaires, ce qui permet de purifier ou d'identifier de l'ARN ou de l'ADN. Ces sondes revêtent également un grand intérêt pour la séparation d'un polynucléotide complémentaire contenu dans une solution amenée au contact de cette molécule supportée, la détection ou la séparation étant mise en oeuvre dans des conditions autorisant leur hybridation mutuelle. Grâce à la formation d'un double brin sur le support il est possible de purifier les protéines donnant lieu à une interaction avec un fragment d'ADN.

D'autres caractéristiques de l'invention apparaîtront encore au cours de la description qui suit d'exemples préférés de construction de molécules supportées conformes à l'invention et d'élongation aux fins d'élaboration d'oligonucléotides. Bien entendu, cette description ne présente aucun caractère limitatif.

## Exemple I

### Fonctionnalisation d'un Support Polyacrylamide

On utilise comme supports, les résines $A_1$, $A_2$ et $A_3$ dont la fonctionnalisation est réalisée comme suit:

Résine $A_1$

On porte à 175°C un mélange de polyacrylmorpholide (gel ENZACRYL K—2, 1 g) et de diamino 1—10 décane (1 g) dans l'éthylène glycol (14 ml) à 175°C pendant une nuit. On ajoute de la glace au milieu réactionnel refroidi, puis on filtre. La résine est lavée successivement par de l'eau (35 ml), de l'acide chlorhydrique 0,1N (35 ml), du carbonate de sodium 0,1N (35 ml), de l'eau (35 ml), du méthanol (70 ml), puis séchée sous vide (1 g). La proportion de fonction amine greffée, déterminée par dosage gisin est de 440 micro M/g pour la résine $A_1$.

Résine $A_2$

On prépare de même, à partir de 3 g du gel ENZACRYL K—2 et de 25 ml d'éthylène diamine, la résine $A_2$.

Résine $A_3$

100 mg du gel ENZACRYL K—2 et 100 mg de spermine, chauffés à 160°C dans 2 ml d'éthylène glycol pendant une nuit, conduisent à 80 mg de résine $A_3$.

1) *Fixation du triazolo-4 O-diméthoxytrityl-5' benzoyl-3'-thymidine (composé 1) sur la résine $A_1$*

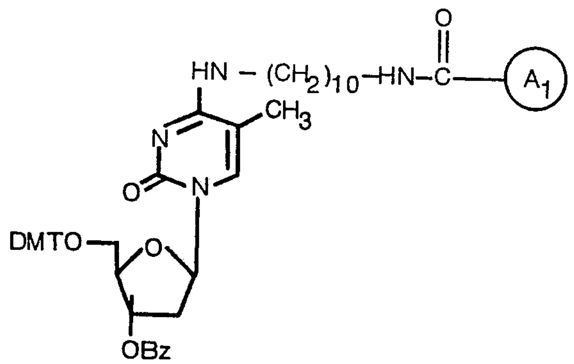

On laisse à température ambiante un mélange de 200 mg de résine fonctionnalisée $A_1$ et de 150 mg du composé *1* dans 4 ml de dioxane. Après 5 jours, la résine est essorée, rincée avec du dioxane ou du méthanol et dosée. Le dosage du diméthoxytrityl (DMT) (par UV à 507 nm) à l'aide de l'acide benzène sulfonique (ABS) indique une substitution de 31 microM/g.

Si on fait réagir la résine avec 85 mg supplémentaires du composé 1, la substitution après 3 jours passe à 63 microM/g. Un traitement à l'ammoniaque concentrée à 50°C (une des conditions de déprotection des séquences d'oligonucléotides) pendant 5 heures n'altère en rien l'accrochage du nucléotide (dosage du groupe diméthoxytrityle). Si l'on chauffe dans l'ammoniaque concentrée à 50°C pendant une nuit, le dosage du DMT montre une perte de capacité de 10%.

2) *Fixation du triazolo-4 O-diméthoxytrityl 5' O-(chloro-2 phényl bêta-cyanoéthyl)phosphate-3' thymidine (composé 2)*
sur la résine $A_1$

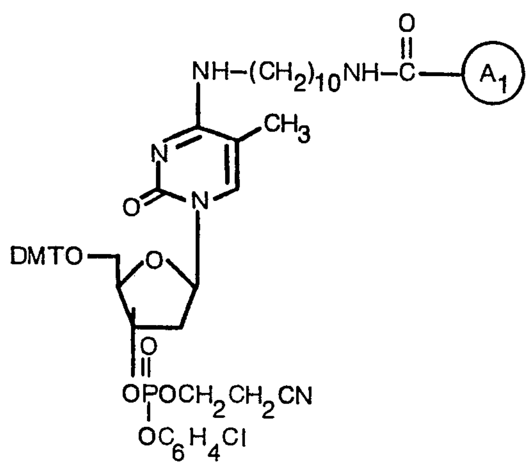

On laisse à température ambiante pendant 20 jours, 220 mg de résine $A_1$, 105 mg du composé *2* dans 2 ml de dioxane. La charge de la résine évaluée par détritylation est de 71 microM/g.

sur la résine $A_2$

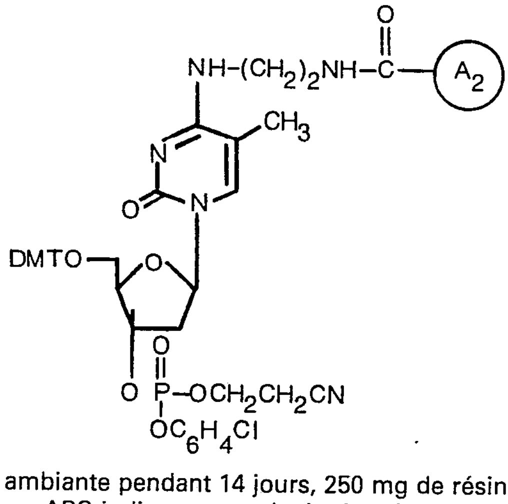

On laisse à température ambiante pendant 14 jours, 250 mg de résine B, 100 mg de composé *2* dans 4 ml de dioxane. Le dosage par ABS indique une substitution de 32 microM/g.

3) *Fixation du triazolo-4 dibenzoyl-2',5' O-(chloro-2 phényl-bêta cyanoéthyl)phosphate-3' uridine (composé 3)* sur la résine $A_1$

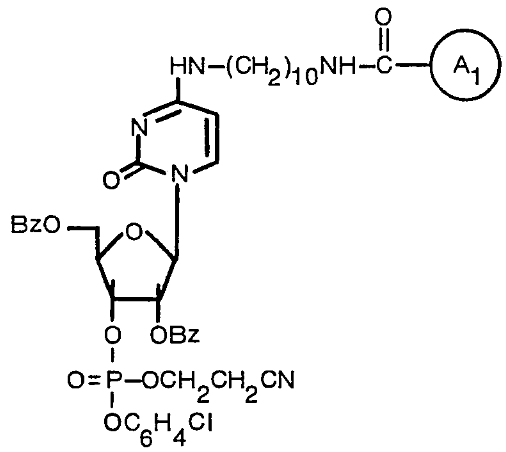

On laisse à température ambiante 200 mg de résine $A_1$, 100 mg du composé *3* dans 4 ml de dioxane pendant 25 jours. Afin de quantifier la substitution de la résine, on effectue un couplage après acétylation des aminés du support (anhydride acétique à 10% dans la pyridine).

Après décyanoéthylation de la résine dans un mélange de triéthylamine/pyridine/eau (1/3/1), coévaporation dans la pyridine, on ajoute 60 mg du composé *1* et 60 mg d'agent de couplage (le triisopropyl benzène-sulfonyl nitrotriazole (IPSNI) dans 300 microlitres de pyridine. Après une heure, la résine est rincée avec de la pyridine, puis un mélange $CH_2Cl_2$/MeOH: 90/10, puis dosée. La détritylation par l'ABS (acide benzènesulfonique), indique une substitution de 29 microM/g en dimère.

4) *Fixation du triazolo-4 O-diméthoxytrityl-5' benzoyl-3' thymidine (composé 1) sur la résine $A_3$*

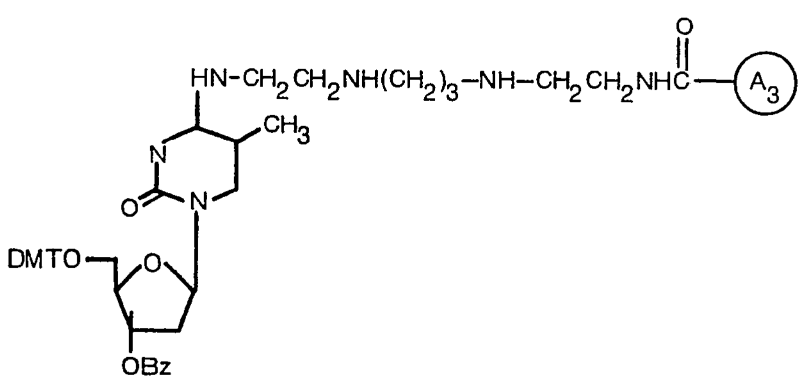

On laisse à température ambiante pendant 13 jours 65 mg de résine $A_3$, 30 mg du composé 1 dans 1 ml de dioxane. La charge de la résine substituée est de 94 microM/g.

Exemple II

Support de Silice

1) *Fixation du triazolo-4 O-diméthoxytrityl-5' benzoyl-3' thymidine composé* 1 *sur la silice "fonctionnalisée"*

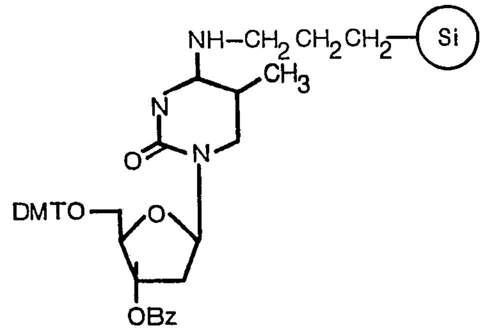

On laisse à température ambiante 500 mg de silice "fonctionnalisée" avec une chaîne aminopropyle (Si-$(CH_2)_3Na_2$) et 200 mg du composé *1* dans 4 ml de dioxane. Après 20 jours, on obtient une substitution de 85 microM/g.

2) *Fixation du triazolo-6 O-diméthoxytrityl-5' benzoyl-3' N-isobutiryl-2 désoxy 2' guanosine (composé 4)*

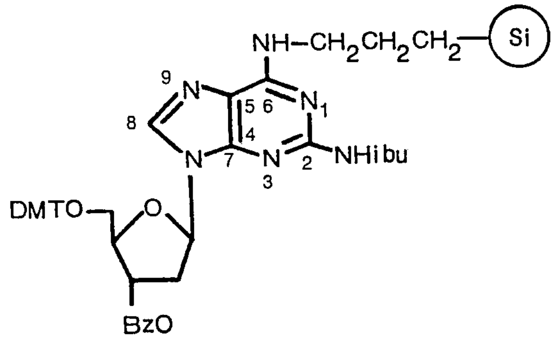

On laisse à température ambiante 500 mg de silice PORASIL fonctionnalisée avec une chaîne aminopropyle et 200 mg du composé *4* dans 4 ml de dioxane. Après 20 jours, on obtient une substitution de 16 microM/g.

Exemple III

Billes de Verre

1) *Fixation du triazolo-4 O-diméthoxytrityl-5' benzoyl-3' thymidine (composé 1) sur un support de verre poreux (en billes)*

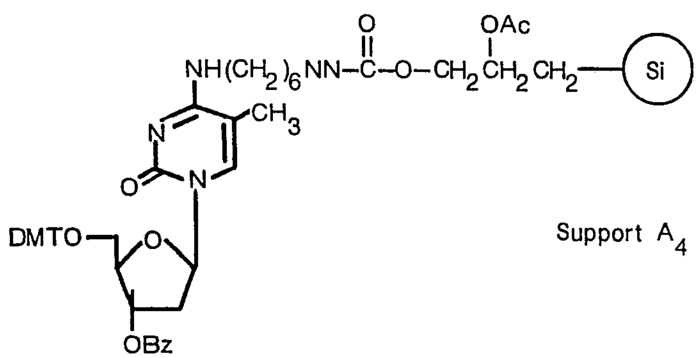

Support $A_4$

On laisse à température ambiante 1 g de billes de verre poreux (de diamètres 500 Angströms) fonctionnalisées par les chaînes apparaissant dans la formule sus-indiquée et commercialisées par la société PIERCE sous la désignation CPG:AALC 500 Angströms, et 310 mg du composé *1* dans 8 ml de dioxanne. Après 16 jours, le support présente une substitution de 22 microM/g.

Afin de vérifier la stabilité de la liaison nucléoside-support, les billes de verre ainsi fonctionnalisées sont chauffées à 50°C pendant une nuit: le dosage du diméthoxytrityl en 5' n'a pas changé; d'autre part, le

contrôle par chromatographie en phase liquide sous haute pression (CLHP) montre l'absence d'hétérocycle dans le filtrat.

Ce support a servi à construire une séquence de "18-mer" avec un rendement moyen de couplage de 85% avec la méthode au phosphotriester, selon la méthode décrite dans la référence bibliographique (4). Après déblocage de tous les groupements protecteurs, le fragement d'ADN lié au support est hydrolysé par l'acide chlorhydrique 2N pendant 5 heures: on retrouve dans le filtrat les 4 bases utilisées dans le rapport convenable (dosage CLHP).

2) *Fixation du triazolo-4 O-diméthoxytrityl-5' benzoyl-2' O-(chloro-2 phényl-bêta-cyanoéthyl)phosphate-3' uridine (composé 5) sur un support de verre poreux*

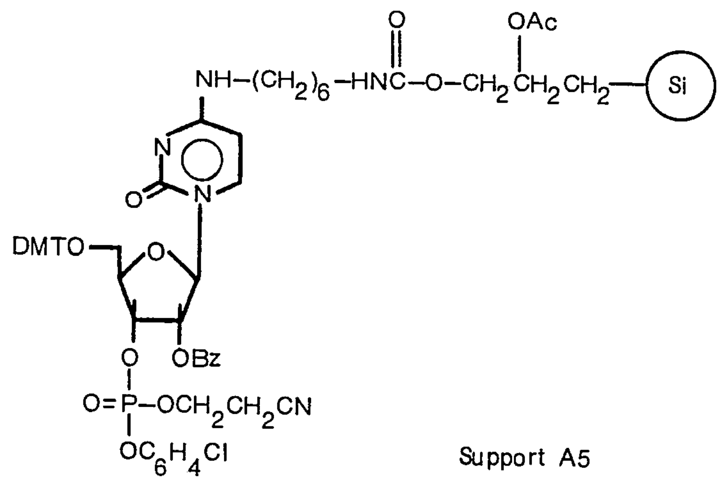

Support A5

On laisse à température ambiante 200 mg de billes de verre CPG/AALL 500 Angströms (PIERCE), 50 mg du composé *5* dans 2 ml de dioxane. Après 17 jours, la substitution est de 10 microM/g.

Exemple IV

Synthese d'un 15-mère à Partir de la Molecule Supportée A5 avec Elongation Dans le Sens 5'—3':5' CCCAGTCACGACGTT 3'

150 mg de support A5 (1,8 µmoles) sont décyanoéthylés par un mélange 1/9 de t-butylamine/pyridine puis couplés avec les trimères suivants: $HO_{CCC}$, $HO_{AGT}$, $HO_{CAC}$, $HO_{GAC}$ et $HO_{GTT_{Tr}}$, chaque étape de couplage étant précédée de la décyanoéthylation de l'oligomère intermédiaire formé. Le support est ensuite soumis aux conditions de déblocage de la séquence: TMGPAO 1 M une nuit puis $NH_4OH$ 50°C 5 h.

Afin de vérifier la présence de l'oligomère synthétisé sur le support, on soumet 50 mg de ces billes à une hydrolyse alcaline (NaOH 2N à 60°C pendant 10 min.). Après purification sur Sephadex G—10 (TEAB 0,05 M), les 2 OD recueillis sont purifiés par chromatographie liquide sous haute pression (CLHP) (Zorbax ODS; gradient d'acétonitrile dans un tampon d'acétate de triéthylammonium $10^{-2}$ M pH 6,5; temps de rétention 10,64 min.). Après détritylation par l'acide acétique 80% pendant 4 h, on obtient 0,23 OD du 15-mère (11% du produit brut) (temps rétention 11,52 min. pour l'oligomère 3' OH et 18,69 min. pour l'oligomère 3' OTr sur HS—5 C18.

Exemple V

Synthèse d'un 15-mère à Partir du Support A5 avec Elongation Dans le Sens 3'—5':5' CCCAGTCACGACGTT 3'

100 mg de support A5 (1,2 µmoles) sont détritylés par l'ABS à 2% puis acétylés par un mélange 1/9 d'anhydride acétique dans la pyridine. La première étape consiste en un couplage 3'—3' entre le support décyanoéthylé et le nucléoside $^{DMT}T$OH (rendement 61%), puis 5 couplages classiques 3'—5' avec $^{DMT}$GT, $^{DMT}$GAC, $^{DMT}$CAC, $^{DMT}$AGT et $^{Tr}$CCC (rdt moyen 80%). Le support est soumis aux conditions de déblocage (TMGPAO 1 M une nuit et $NH_4OH$ 50°C 5 h). L'oligomère est décroché du support pour caractérisation, par hydrolyse alcaline (NaOH 2N 10 min.). 50 mg de ces billes conduisent à 1,6 OD après purification sur Sephadex G—10. Après purification par CLHP et détritylation on obtient 0,35 OD du 15-mère (22% du produit brut) (HS—5 C18; temps rétention 11,57 min. pour le 3'OH et 17,25 min. pour le 3' OTr).

Exemple VI

Synthèse d'un 34-mère Comportant un Site de Restriction RsaI à Partir du Support A4

La synthèse a été réalisée mutuellement par la méthode au phosphotriester (sur 100 mg de billes) et automatiquement par la méthode au phosphoramidite (à partir de 30 mg de billes). La présente d'un site de restriction RsaI dans la séquence permet de contrôler la synthèse. La première étape consiste à marquer les

extrémités 5' du support (polynucléotide kinase) puis à faire agir l'enzyme de restriction RsaI en présence d'un 16-mère complémentaire synthétisé de façon classique. Sur le gel d'électrophorèse des surnageants, on note la présence pour les deux supports d'une bande correspondant à un 9-mère résultant de la coupure enzymatique.

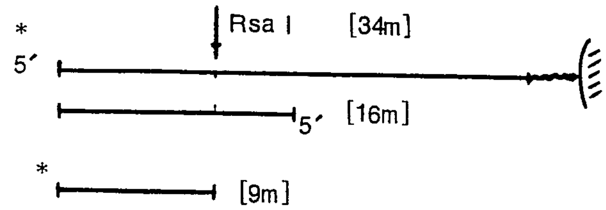


HEPTAMERE 2: 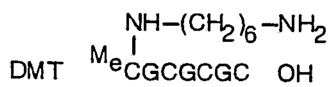

Après synthèse en phase liquide selon la méthode au phosphotriester de l'heptamère "activé" DMTTtTGCGCGC puis substitution par l'hexanediamine, l'oligomère fonctionnalisé (118 mg; 32 umoles) est débloqué selon les conditions classiques. Après purification sur Sephadex G—10, et CLHP (Zorbax ODS; tps de rétention 11,40 min.), on obtient 2,3 mg de l'heptamère *2* ayant l'hydroxyle 5' diméthoxytrilé.

SUPPORT 1: 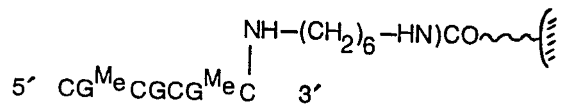

Aux 200 mg de Sepharose 4B activée lavés selon les conditions décrites (HCl $10^{-3}$ M), on ajoute 1,2 mg (0,54 umoles) de l'heptamère *1* en solution dans un ml de tampon *a* de réaction ($NaHCO_3$ 0,1 M et NaCl 0,5 M). Après 1 h à température ambiante et la nuit à 4°C, le support est essoré sur fritté et lavé avec le tampon *a*. Les fonctions activées du support n'ayant pas réagi sont bloquées par un tampon *b* (Tris-HCl 0,1 M; pH 8; 1 ml pendant 1 h à 4°C). Le support est ensuite rincé par un cycle de tampons *c* et *d* (*c*: Tris 0,1 M et NaCl 0,5 M pH 8. *d*: acétate de sodium 0,1 M et NaCl 0,5 M pH 4).

SUPPORT 2: 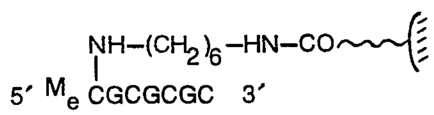

Aux 200 mg de Sepharose 4B activée lavés selon les conditions décrites on ajoute 1,1 mg (0,50 moles) de l'heptamère *2* en solution dans 1 ml de tempon *a*. Après 1 h à température ambiante et la nuit à 4°C, le support *2* est traité comme précédemment (cf support 1).

La substitution des supports *1* et *2* peut être évaluée par dosage en UV à 507 nm du cation DMT+ (détritylation par l'acide trichloracétique). L'incorporation est de 90% environ, soit 2,4 moles/g pour le support *1* et 2,2 mole/g pour le support *2*.

Verification-Controle

Les supports *1* et *2* (10 à 20 mg essorés) sont soumis à une hydrolyse acide (HCl 2N à 80°C), ainsi que le support activé de départ *3* et ce même support activé soumis à un hexamère non fonctionnalisé $CG^{ME}CGCG$ *4*. Ceux-ci serviront de blancs à notre test. L'analyse par CLHP des hydrolysats indique la présence des bases cytosine et guanine pour les supports *1* et *2* et une faible association (10%) dans le cas du support *4* qui peut être due à une absorption de l'hexamère sur le support.

On soumet les supports *1* et *2* à l'action de la phosphodiestérase I (Crotalus durcissus): 1 mg de support dans 100 ul de tampon (0,025 M Tris et 0,05 $MgCl_2$ pH 9) et 10 ul d'enzyme (10 ug) portés 3 h à 37°C puis une nuit à température ambiante.

L'analyse des surnageants bruts par CLHP sur Partisil PXS 10/25 SAX (isochratique; $10^{-2}$ M $KH_2PO_4$ et $10^{-1}$ M de KCl, pH = 5) indique pour les supports *1* et *2* la présence de pdC et pdG (tps de rétention de 10,7 et 18,4 min. identiques aux témoins) alors que le test est négatif pour le support *4*.

Bibliographie


(1) H. Koster et Coll., Tetrahedron, *40*, 103, 1984.
(2) H. Caruthers et Coll., Tetrahedron Letters, *21*, 719, 1980.
(3) M. J. Gait et Coll., Nucleic Acids Research, *10*, 6243, 1982.
(4) K. Itakura et Coll., Nucleic Acids Research, *8*, 5507, 1980.
(5) A. Rosenthal et Coll. Tetrahedron Letters, *24*, 1691, 1983.
(6) C. K. Brush et Coll., Nucleic Acids Research, *10*, 6295, 1982.

(7) C. B. Reese et Coll., Tetrahedron Letters, *21*, 2265, 1980.
(8) W. L. Sung, J. Org. Chem., *47*, 3623, 1982.
(9) C. B. Reese et Coll., Nucleic Acids Research, Symposium Series 7, Koster Editor, p. 5.
(10) C. B. Reese et Coll., Tetrahedron Letters, *22*, 4755, 1981.
(11) R. A. Jones et Coll., Tetrahedron Letters, *40*, 3, 1984.
(12) G. R. Gough et Coll., Tetrahedron Letters, *24*, 5317, 1983.
(13) R. Gumport et Coll. The Enzymes vol. XV, p. 31. P. D. Boyer Edit (1982) Academic Press.
(14) R. Wu, L. Grossman et K. Moldave, Methods in Enzymology, vol. 100, p. 38, Academic Press (1983).

**Revendications**

1. Oligonucléotides ou polynucléotides fixés sur un support solide, caractérisés en ce qu'ils comportent au moins une structure de formule:

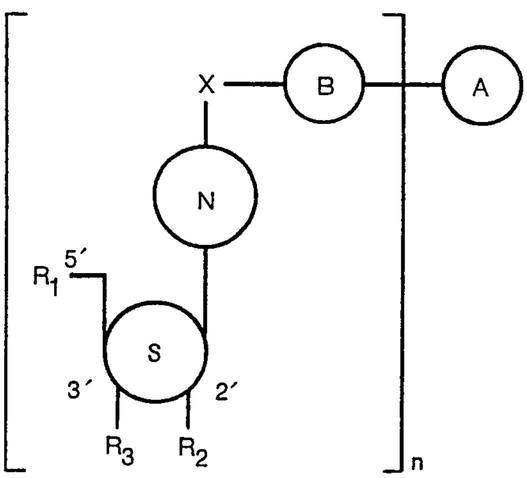

dans laquelle:

N représente une base, telle que uracile, méthyl-5-uracile, guanine, xanthine, méthyl-5-cytosine, cytosine, amino-2-adénine, hypoxanthine;

S représente un motif ribose ou désoxy-2'-ribose avec $R_1$ représentant un groupe OH, phosphate ou encore oligo ou polynucléotide, $R_2$ un atome d'hydrogène ou un groupe OH et $R_3$ un groupe OH, phosphate ou encore oligo ou polynucléotide, les chiffres (2'), (3') et (5') correspondant aux sommets, numérotés de façon classique, des sucres en question, les séquences de l'oligo ou polynucléotide pouvant être complémentaires et formant un double brin ou l'oligonucléotide fixé sur l'amorce pouvant être replié en boucle en formant un double brin et étant fixé sur l'amorce par l'un des nucléotides de la boucle,

X représente un groupe divalent NH ou O fixé sur la base N au niveau des sommets 4 ou 6 respectivement pour une base pyrimidique et pour une base purique,

B représente une chaîne carbonée de 4 à 20 atomes de carbone,

A représente un support solide,

n représente le nombre de chaînes fixées sur un même support A, ces chaînes pouvant être différentes les unes des autres et fixées par des bras identiques ou différents sur le support, sous réserve que lorsque N représente une adénine et l'un des groupes $R_1$ et $R_3$ représente un groupe —OH et X représente NH, B soit différent d'un radical dodécylamine.

2. Oligonucléotides ou polynucléotides selon la revendication 1, caractérisés en ce que B représente une chaîne carbonée de 4 à 20 atomes de carbone, dont certains peuvent, le cas échéant, être remplacés par des hétéroatomes, tels que O, N ou S.

3. Oligonucléotides ou polynucléotides selon la revendication 1, caractérisés en ce que X—B correspond à une chaîne dérivée d'une chaîne bifonctionnelle telle que l'éthylène diamine, le diamino 1—6 hexane, le diamino 1—6 décane, la spermine, des diéthylène-glycols ou dialcoylèneglycols.

4. Oligonucléotides ou polynucléotides selon la revendication 1, caractérisés en ce que B renferme un groupe M'' choisi parmi une fonction amide, éther, sulfure, uréthane ou encore un groupe —NH-ligand, ce groupe M'' résultant de la réaction d'une part du groupe réactif M, situé à l'extrémité d'un bras fixé à un nucléotide ou à un nucléoside, avec d'autre part un groupe réactif M' fixé sur le support solide A.

5. Oligonucléotides ou polynucléotides fixés sur un support A tels qu'obtenus par allongement de la chaîne nucléotidique en positions 3' et 5' d'une amorce répondant à la structure:

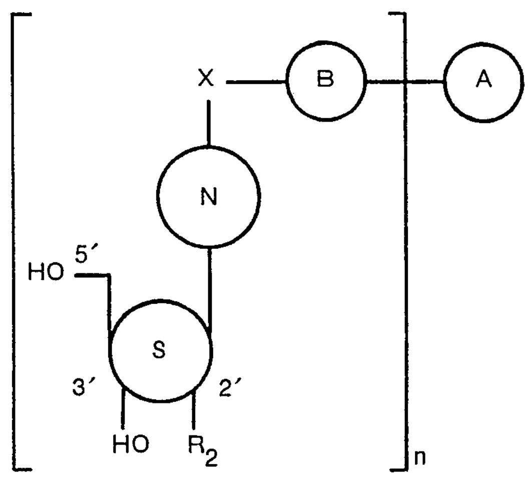

dans laquelle:

N représente une base, telle que uracile, méthyl-5-uracile, guanine, xanthine, méthyl-5-cytidine, cytidine, thymidine, amino-2-adénine, inosine;

S représente un motif ribose ou désoxy-2'-ribose avec $R_2$ représentant un atome d'hydrogène ou un groupe OH;

X représente un groupe divalent NH ou O fixé sur la base N au niveau des sommets 4 ou 6 respectivement pour une base pyrimidique et pour une base purique,

B représente une chaîne carbonée de 4 à 20 atomes de carbone,

A représente un support solide,

n représente le nombre de chaînes fixées sur un même support A, ces chaînes pouvant être différentes les unes des autres et fixées par des bras identiques ou différents sur le support, l'allongement des chaînes étant suivi le cas échéant d'une déprotection des nucléotides ou des nucléosides.

6. Procédé de préparation d'oligonucléotides ou de polynucléotides selon l'une des revendications 1 à 5, caractérisé en ce qu'il comprend la réaction

a) du sommet 6 ou 4 respectivement d'une base purique ou pyrimidique d'un nucléoside ou d'un nucléotide, ce sommet étant activé par un dérivé hétérocyclique ou par l'intermédiaire d'un chlorure d'acide, avec

b) un groupe terminal $NH_2$ ou OH d'un bras B, tel que défini dans la revendication 1, fixé à un support solide,

c) la synthèse de la chaîne nucléotidique en 3' et/ou 5' selon les techniques classiques, suivie le cas échéant d'une déprotection des nucléotides et nucléosides.

7. Procédé de préparation d'oligonucléotides ou de polynucléotides selon l'une des revendications 1 à 5, caractérisé en ce qu'il comprend:

1) la réaction du sommet activé 6 ou 4 respectivement d'une base purique ou pyrimidique située dans une position quelconque dans un oligonucléotide synthétisé au préalable, avec un groupe terminal réactif d'une chaîne bifonctionnelle, puis

2) la réaction du nucléoside ou du nucléotide fonctionnalisé obtenu avec un support solide approprié.

8. Procédé selon la revendication 7, caractérisé en ce qu'on effectue la réaction illustrée par le schéma suivant:

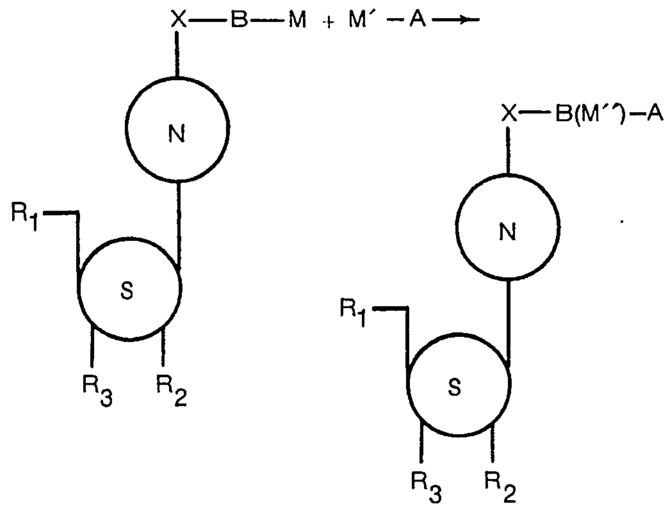

dans lequel $R_1$ à $R_3$, S, N, X, B, M'' et A sont tels que définis ci-dessus, M est choisi parmi $—NH_2$, —SH, —OH, —COOH ou NH-ligand, et M' représente, respectivement, compte tenu des significations ci-dessus de *M* un groupe —COOH, —SH, —OH ou $NH_2$, B (M'') signifiant que B renferme le groupe fonctionnel M'' qui résulte de la réaction de M avec M' défini plus haut.

9. Procédé selon l'une des revendications 6 à 8 caractérisé en ce que le sommet de la base purique ou pyrimidique a été activé par fixation d'un dérivé hétérocyclique du type triazole, nitrotriazole ou tétrazole ou par l'intermédiaire d'un chlorure d'acide.

10. Procédé de préparation d'oligonucléotides ou de polynucléotides selon les revendications 7 ou 8, caractérisé en ce qu'on fixe un oligonucléotide double brin fonctionnalisé en position 4 ou 6 respectivement d'une base pyrimidique ou purique comme défini dans la revendication 8, sur un support M'A.

11. Sondes, pour la détection de séquences complémentaires, la séparation d'un polynucléotide complémentaire contenu dans une solution, ou encore la purification de protéines, caractérisées en ce qu'elles sont constituées à partir d'un oligonucléotide ou d'un polynucléotide mono brin ou double brin fixé sur un support selon l'une quelconque des revendications 1 à 5.

**Patentansprüche**

1. Oligonukleotide oder Polynukleotide, die auf einen festen Träger fixiert sind, dadurch gekennzeichnet, daß sie mindestens eine Struktur mit der Formel besitzen:

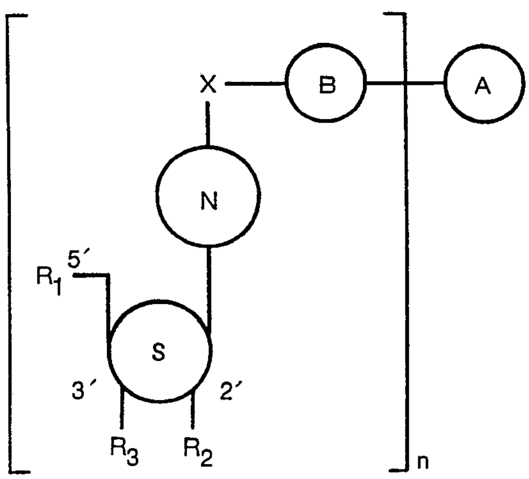

in der:

N eine Base darstellt, wie Uracil, Methyl-5-uracil, Guanin, Xanthin, Methyl-5-cytosin, Cytosin, Amino-2-adenin, Hypoxanthin,

S eine Ribose- oder Desoxy-2'-ribosegruppe darstellt, wobei $R_1$ eine OH-, Phosphat- oder weitere Oligo- oder Polynukleotidgruppe darstellt, $R_2$ ein Wasserstoffatom oder eine OH-Gruppe und $R_3$ eine OH-,

Phosphat- oder weitere Oligo- oder Polynukleotidgruppe, die Ziffern (2'), (3') und (5') den Positionen der fraglichen Zucker entsprechen, die in klassischer Weise numeriert sind, die Sequenzen des Oligo- oder Polynukleotids können komplementär sein und einen Doppelstrang bilden oder das Oligonukleotid, das auf das Anfangsstück fixiert ist, kann in eine Schleife gefaltet sein wobei es einen Doppelstrang bildet und durch eines der Nukleotide der Schleife auf das Anfangsstück fixiert ist,

X eine divalente NH- oder O-Gruppe darstellt, die an die Base N fixiert ist in Höhe der Positionen 4 oder 6 entsprechend für eine Pyrimidinbase oder für eine Purinbase.

B eine Kohlenstoffkette darstellt mit 4 bis 20 Kohlenstoffatomen,

A einen festen Träger darstellt,

n die Zahl der fixierten Ketten auf einem Träger A darstellt, diese Ketten können voneinander verschieden sein und durch identische oder verschiedene Arme auf dem Träger fixiert sein, unter Vorbehalt, daß wenn N ein Adenin darstellt und eine der Gruppen $R_1$ und $R_3$ eine OH-Gruppe darstellt und X NH darstellt, B von einem Dedecylaminradikal verschieden ist.

2. Oligonukleotide oder Polynukleotide gemäß Anspruch 1, dadurch gekennzeichnet, daß B eine Kohlenstoffkette darstellt mit 4 bis 20 Kohlenstoffatomen, von denen gegebenenfalls einige durch Heteroatome wie O, N oder S ersetzt sind.

3. Oligonukleotide oder Polynukleotide gemäß Anspruch 1, dadurch gekennzeichnet, daß X—B einer Kette entspricht, die von einer bifunktionellen Kette abgeleitet ist, wie Ethylendiamin, Diamino-1,6-hexan, Diamino-1,6-decan, Spermin, Diethylenglykole oder Dialkoylenglykole.

4. Oligonukleotide oder Polynukleotide gemäß Anspruch 1, dadurch gekennzeichnet, daß B eine Gruppe M'' einschließt, die ausgewählt ist aus einer Amid-, Ether-, Sulfid-, Urethanfunktion oder ferner einer —NH-Ligand-Gruppe, diese Gruppe M'' resultiert aus der Reaktion einerseits der reaktiven Gruppe M, die am Ende eines Arms liegt, der an ein Nukleotid oder an ein Nukleosid fixiert ist, mit andererseits einer reaktiven Gruppe M', die auf den festen Träger A fixiert ist.

5. Oligonukleotide oder Polynukleotide, die auf einen Träger A fixiert sind, erhalten durch Verlängern der Nukleotidkette in den Positionen 3' und 5' von einem Stück entsprechend der Struktur:

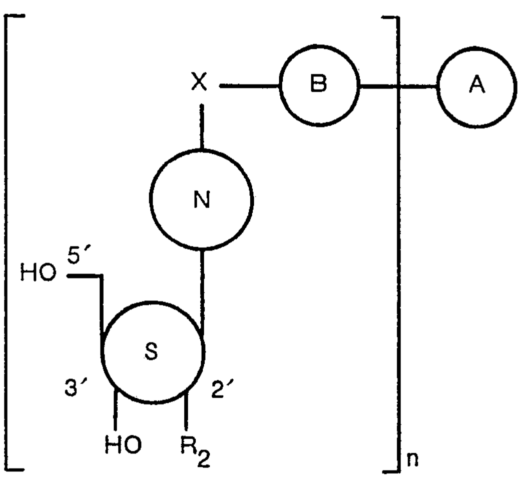

in der

N eine Base darstellt wie Uracil, Methyl-5-uracil, Guanin, Xanthin, Methyl-5-cytidin, Cytidin, Thymidin, Amino-2-adenin, Inosin,

S eine Ribose- oder Desoxy-2'-ribosegruppe darstellt, wobei $R_2$ ein Wasserstoffatom oder eine OH-Gruppe darstellt,

X eine divalente NH- oder O-Gruppe darstellt, die an der Base N fixiert ist in Höhe der Positionen 4 oder 6 entsprechend für eine Pyrimidinbase oder für eine Purinbase,

B eine Kohlenstoffkette darstellt mit 4 bis 20 Kohlenstoffatomen,

A einen festen Träger darstellt,

n die Zahl der Ketten darstellt, die auf demselben Träger A fixiert sind, diese Ketten können voneinander verschieden sein und durch identische oder verschiedene Arme auf dem Träger fixiert sein, die Verlängerung der Ketten wird gegebenenfalls durch eine Deprotektion der Nukleotide oder der Nukleoside erreicht.

6. Verfahren zur Herstellung von Oligonukleotiden oder von Polynukleotiden gemäß einem der Ansprüche 1 bis 5, dadurch gekennzeichnet, daß es die Reaktion umfaßt:

a) der Position 6 oder 4 entsprechend einer Purinbase oder Pyrimidinbase eines Nukleosids oder eines Nukleotids, wobei diese Position aktiviert ist durch ein heterocyclisches Derivat oder durch Vermittlung eines Säurechlorids, mit

b) einer $NH_2$- oder OH-Endgruppe von einem Arm B, wie im Anspruch 1 definiert, der an einen festen Träger fixiert ist,

c) die Synthese der Nukleotidkette bei 3' und/oder 5' gemäß den klassischen Techniken,

gegebenenfalls gefolgt von einer Deprotektion der Nukleotide und Nukleoside.

7. Verfahren zur Herstellung von Oligonukleotiden oder von Polynukleotiden gemäß einem der Ansprüche 1 bis 5, dadurch gekennzeichnet, daß es umfaßt:

1) die Reaktion der aktivierten Position 6 oder 4 entsprechend einer Purinbase oder Pyrimidinbase, die an irgendeiner Stelle in einem Oligonukleotid liegt, das zuvor synthetisiert ist, mit einer reaktiven Endgruppe einer bifunktionellen Kette, dann

2) die Reaktion des erhaltenen funktionalisierten Nukleosids oder Nukleotids mit einem geeigneten festen Träger.

8. Verfahren gemäß Anspruch 7, dadurch gekennzeichnet, daß die durch das folgende Schema erläuterte Reaktion durchgeführt wird:

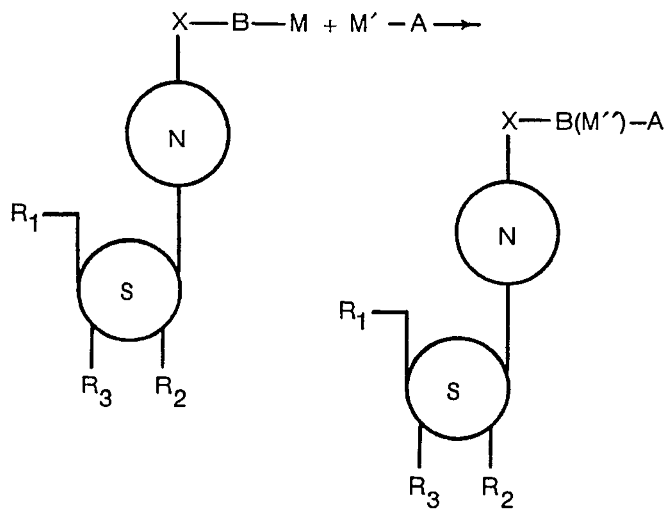

in der $R_1$ bis $R_3$, S, N, X, B, M'' und A wie oben definiert sind, M ist gewählt aus —$NH_2$, —SH, —OH, —COOH oder NH-Ligand, und M' stellt entsprechend, unter Berücksichtigung der obigen Bedeutungen von M, eine —COOH—, —SH—, —OH— oder $NH_2$-Gruppe dar, B (M'') bedeutet, daß B die funktionelle Gruppe M'' einschließt, die aus der Reaktion von M mit M' resultiert, die weiter oben definiert wurde.

9. Verfahren gemäß einem der Ansprüche 6 bis 8, dadurch gekennzeichnet, daß die Position der Purin- oder Pyrimidinbase aktiviert wurde durch Fixierung eines heterocyclischen Derivats vom Typ Triazol, Nitrotriazol oder Tetrazol oder durch Vermittlung eines Säurechlorids.

10. Verfahren zur Herstellung von Oligonukleotiden oder von Polynukleotiden gemäß den Ansprüchen 7 oder 8, dadurch gekennzeichnet, daß ein Oligonukleotid-Doppelstrang, der in Position 4 oder 6 entsprechend einer Pyrimidinbase oder Purinbase funktionalisiert ist wie im Anspruch 8 definiert, auf einen Träger M'A fixiert wird.

11. Sonden für den Nachweis von komplementären Sequenzen, die Trennung eines komplementären Polynukleotids, das in einer Lösung enthalten ist, oder ferner für die Reinigung von Proteinen, dadurch gekennzeichnet, daß sie aufgebaut sind ausgehend von einem Oligonukleotid- oder von einem Polynukleotid in Form eines Einzelstrangs oder Doppelstrangs, der auf einen Träger gemäß einem der Ansprüche 1 bis 5 fixiert ist.

**Claims**

1. Oligonucleotides or polynucleotides fixed on a solid support, characterised in that they comprise at least a structure of formula:

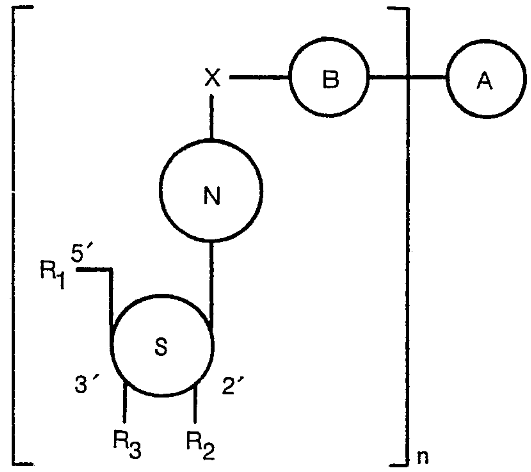

in which:

N represents a base such as uracil, methyl-5-uracil, guanine, xanthine, methyl-5-cytosine, cytosine, amino-2-adenine, hypoxanthine,

S represents a ribose or a deoxy-2'-ribose moiety with $R_1$ representing an OH group, a phosphate or even an oligo- or polynucleotide, $R_2$ a hydrogen atom or an OH group and $R_3$ an OH group, a phosphate or even an oligo- or polynucleotide, the numbers (2'), (3') and (5') corresponding to the positions of the sugar residues in question, numbered in the accepted manner, the oligo- or polynucleotide sequences being or not complementary and forming a double strand or the oligonucleotide fixed on the primer may be bent into a loop by forming a double strand and being fixed on the primer by one of the nucleotides of the loop,

X represents a NH or O divalent group fixed on the base N at the level of the positions 4 or 6 respectively for a pyrimidine base and for a purine base,

B represents a carbon chain of 4 to 20 carbon atoms, and

A represents a solid support,

n represents the number of chains fixed on a same support A, said chains may be different from one another and fixed by identical or different arms on the support, with the proviso that when N represents an adenin and one of the group $R_1$ and $R_3$ represent an —OH group and X represents NH, B be different from a dodecylamine radical.

2. Oligonucleotides or polynucleotides according to claim 1, characterised in that B represents a carbon chain of 4 to 20 carbon atoms, some of which may be replaced by heteroatoms such as O, N or S.

3. Oligonucleotides or polynucleotides according to claim 1, characterised in that X—B corresponds to a chain derived from a bifunctional chain such as ethylene diamine, hexane 1,6-diamine, decane 1,6-diamine, spermine, diethylene-glycols or dialkylene glycols.

4. Oligonucleotides or polynucleotides according to claim 1, characterised in that B contains a group M'' chosen from among an amide, ether, sulfur, urethane function, or again a NH-ligand group, the group M'' resulting from the reaction of a reactive grouping M, situated at the extremity of a spacer arm attached to a nucleoside or nucleotide, with a reactive group M' attached to the solid support A.

5. Oligonucleotides or polynucleotides fixed on a support A such as obtained by elongating the nucleotide chain in the 3' and 5' positions of a primer having the structure:

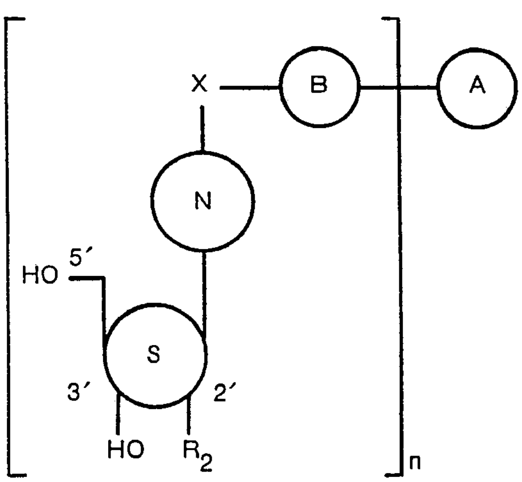

wherein

N represents a base, such as uracil, methyl-5-uracil, guanine, xanthine, methyl-5-cytidine, cytidine, thymidine, amino-2-adenine, inosine,

S represents a ribose or a deoxy-2'-ribose with $R_2$ representing an hydrogen atom or an —OH group,

X represents a NH or O divalent group fixed on the base N at the level of the positions 4 or 6 respectively for a pyrimidine base and for a purine base,

B represents a carbon chain of 4 to 20 carbon atoms, and

n represents the number of chains fixed on a same support A, said chains may be different from one another and fixed by identical or different arms on the support, the elongation of the chains being optionally followed by a deprotection of the nucleotides or nucleosides.

6. Process for the preparation of oligonucleotides or polynucleotides according to any one of claims 1 to 5, characterised in that it comprises the reaction:

a) of position 6 or 4 respectively of a purine or pyrimidine base of a nucleoside or nucleotide, this position being activated by a heterocyclic derivative or by means of an acid chloride with

b) a terminal $NH_2$ or OH group of an arm B, as defined in claim 1, attached to a solid support,

c) the synthesis of the nucleotidic chain in 3' and/or 5' according to the usual method optionally followed by a deprotection of the nucleotides and nucleosides.

7. Process for the preparation of oligonucleotides or polynucleotides according to any one of claims 1 to 5, comprising:

1) the reaction of the activated position 6 or 4 of a purine base or pyrimidine base respectively located at any position in a previously synthesized oligonucleotide, with a reactive end group of a bifunctional chain, then

2) the reaction of the functionalized nucleoside or nucleotide obtained with an appropriate solid support.

8. Process according to claim 7, characterised in that the reaction illustrated in the following scheme is carried out:

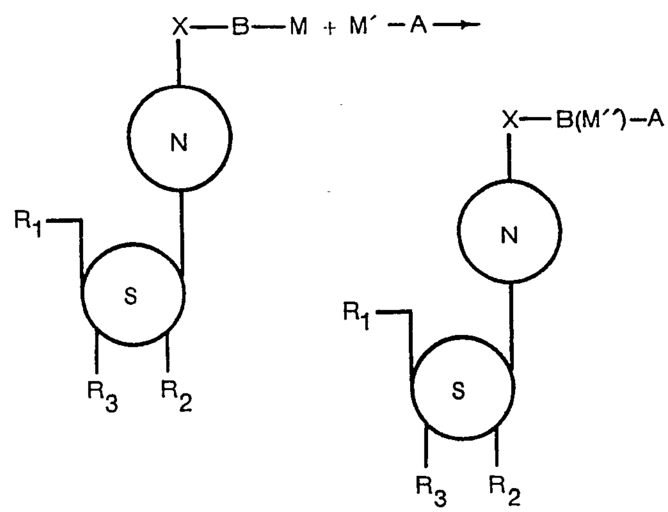

in which $R_1$ to $R_3$, S, N, X, B, M'' and A have the meanings defined above, M is chosen from among $—NH_2$, —SH, —OH, —COOH or —NH-ligand, and M' represents, respectively, a —COOH, —SH, —OH or $NH_2$ group, depending on the meaning of M above, B (M'') meaning that B comprises the functional group M'' which results from the reaction of M with M' as defined above.

9. Process according to one of the claims 6 to 8, characterised in that the position of the purine or pyrimidine base was activated by attachment of a heterocyclic derivative of the triazole, nitrotriazole or tetrazole type or by the intermediary of an acid chloride.

10. Process for preparing oligonucleotides or polynucleotides according to claim 7 or 8, wherein a functionalized double strand oligonucleotide is fixed at position 4 or 6 of a purine or pyrimidine base respectively as defined in claim 8, on support M'A.

11. Probes for detecting complementary sequences, the separation of a complementary polynucleotide contained in a solution, or again the purification of proteins, characterised in that they are elaborated from a single strand or a double strand oligonucleotide or polynucleotide according to any one of claims 1 to 5.